(19) Europäisches Patentamt / European Patent Office / Office européen des brevets

(11) **EP 2 868 669 A1**

(12) **DEMANDE DE BREVET EUROPEEN**

(43) Date de publication:
06.05.2015 Bulletin 2015/19

(21) Numéro de dépôt: 14200048.8

(22) Date de dépôt: 08.04.2004

(51) Int Cl.:
*C08B 37/00* (2006.01)  *A61K 47/36* (2006.01)
*A61L 31/04* (2006.01)  *A61L 27/52* (2006.01)
*A61K 6/097* (2006.01)  *A61L 27/20* (2006.01)
*A61L 27/50* (2006.01)  *C08B 37/08* (2006.01)
*C08J 3/24* (2006.01)  *C08L 5/08* (2006.01)

(84) Etats contractants désignés:
**DE ES FR GB IT**

(30) Priorité: **10.04.2003 FR 0304444**

(62) Numéro(s) de document de la (des) demande(s) initiale(s) en application de l'article 76 CBE:
**10181594.2 / 2 289 945**
**04742458.5 / 1 611 160**

(71) Demandeur: **ALLERGAN INDUSTRIE**
**74370 Pringy (FR)**

(72) Inventeur: **Lebreton, Pierre**
**74000 Annecy (FR)**

(74) Mandataire: **Hoffmann Eitle**
**Patent- und Rechtsanwälte PartmbB**
**Arabellastraße 30**
**81925 München (DE)**

Remarques:
Cette demande a été déposée le 23-12-2014 comme demande divisionnaire de la demande mentionnée sous le code INID 62.

(54) **HYALURONATE DE SODIUM SE PRÉSENTANT SOUS LA FORME DE DEUX MASSES MOLÉCULAIRES DIFFÉRENCIÉES RÉTICULÉ ET HYDROGEL MONOPHASIQUE INJECTABLE LE CONTENANT**

(57) La présente invention a pour objet : - un procédé de réticulation d'au moins un polymère, choisi parmi les polysaccharides et leurs dérivés, mis en oeuvre en solvant aqueux par action d'un quantité efficace et non excessive d'au moins un agent de réticulation, **caratérisé en ce qu**'il est mis en oeuvre sur un mélange renfermant au moins un polymère de faible masse moléculaire et au moins un polymère de forte masse moléculaire ; - un procédé de préparation d'un hydrogel monophasique injectable d'au moins un polymère réticulé choisi parmi les polysaccharides et leurs dérivés ; - les polymères réticulées et hydrogels monophasiques injectables, susceptibles d'être respectivement obtenus par chacun desdits procédés.

EP 2 868 669 A1

**Description**

La présente invention a pour objet :

- un procédé de réticulation, original, d'au moins un polymère, choisi parmi les polysaccharides et leurs dérivés,
- un procédé de préparation d'un hydrogel monophasique injectable d'au moins un tel polymère,
- les polymères réticulés et hydrogels monophasiques injectables, susceptibles d'être respectivement obtenus par chacun desdits procédés.

Les hydrogels en cause, à base desdits polymères réticulés, ont de nombreux débouchés, notamment comme matériau de comblement en chirurgies réparatrice, esthétique, dentaire, en ophtalmologie, en orthopédie... comme produit empêchant les adhésions de tissus, en chirurgie générale, en urologie... Lesdits hydrogels conviennent notamment pour la réparation des cordes vocales. Les débouchés indiqués ci-dessus, de façon nullement limitative, pour ce type de produits, sont familiers à l'homme du métier.

L'invention résulte d'un réel effort d'optimisation de la mise en oeuvre de la réticulation des polymères en cause, en vue d'obtenir des hydrogels monophasiques injectables, particulièrement intéressants en référence au compromis ci-après : propriétés mécaniques et rémanence, d'une part, injectabilité (avec des forces d'injection et des diamètres d'aiguilles d'injection, acceptables), d'autre part.

On précise ici que le qualificatif injectable, employé dans le présent texte, tant en référence aux hydrogels de l'art antérieur qu'aux hydrogels de l'invention, signifie injectable manuellement au moyen de seringues munies d'aiguilles classiques (d'un diamètre compris entre 0,1 et 0,5 mm). Dans le cadre de la présente invention, il est notamment possible de formuler des hydrogels injectables au travers d'aiguilles hypodermiques de 30 G½, 27 G½, 26 G½, 25 G.

Selon l'art antérieur, on a déjà préparé, à partir de polysaccharides et de leurs dérivés - notamment à partir de sels d'acide hyaluronique - pas, peu ou fortement réticulés, des hydrogels, notamment des hydrogels injectables.

En référence au problème spécifique de l'injectabilité, des compositions biphasiques ont été proposées, dont notamment la phase continue est à base de tels hydrogels. Ladite phase continue sert de plastifiant, de véhicule d'injection à une phase dispersée. Cette phase dispersée est plus ou moins solide, plus ou moins différenciée de la phase continue. Ainsi :

- dans la demande EP-A-0 466 300, les compositions biphasiques décrites sont constituées de deux phases - continue, dispersée - biorésorbables et se présentent sous la forme de boues. Lesdites deux phases sont avantageusement préparées à partir de fibres de Hylan (acide hyaluronique naturel modifié chimiquement in situ dans le but de faciliter son extraction des tissus),
- dans la demande WO-A-96 337 51, les compositions biphasiques décrites présentent également deux phases biorésorbables, mieux séparées, la phase dispersée étant constituée de fragments insolubles d'un hydrogel de polymère (choisi parmi l'acide hyaluronique et ses sels), fortement réticulé ;
- dans la demande WO-A-00 014 28, les compositions biphasiques décrites renferment une phase dispersée, non biorésorbable (particules d'au moins un hydrogel d'un (co)polymère obtenu par polymérisation et réticulation de l'acide acrylique et/ou de l'acide méthacrylique et/ou d'au moins un dérivé desdits acides), en suspension dans une solution aqueuse d'un polymère choisi parmi les protéines, les polysaccharides et leurs dérivés, réticulé ou non.

Ces systèmes biphasiques ne sont pas pleinement satisfaisants dans la mesure où l'on peut justement craindre des à-coups à l'injection et surtout après injection, une disparition plus rapide de la phase continue (pas ou faiblement réticulée) et donc une perte au moins partielle de l'effet, notamment de comblement, recherché.

On a donc également proposé, en parallèle, des hydrogels monophasiques, élaborés à partir des mêmes types de polymères.

Dans les demandes WO-A-98 356 39 et WO-A-98 356 40, le produit en cause n'est pas un hydrogel injectable mais un produit de consistance solide. Dans lesdites demandes, on décrit en fait des implants oculaires, utilisés pour combler temporairement un vide créé chirurgicalement. Dans le brevet US-A-4 716 154, l'hydrogel élaboré est proposé comme substitut au corps vitré. Le polymère en cause (hyaluronate de sodium) est très peu réticulé, pour l'obtention d'un hydrogel injectable. Dans la demande WO-A-02 057 53, l'hydrogel monophasique décrit est chargé d'un antiseptique, efficace pour le protéger, après implantation, vis-à-vis des radicaux libres. Dans la demande WO-A-02 063 50, un procédé est décrit, apte à générer ce type d'hydrogel, très homogène dans sa masse.

Tous ces hydrogels monophasiques ont été obtenus à partir de polymères de forte masse moléculaire, réticulés avec intervention d'une quantité efficace et non excessive d'au moins un agent de réticulation, en solvant aqueux,

Au vu de cet art antérieur, les inventeurs ont souhaité améliorer l'efficacité de la réticulation du polymère en cause de façon à notamment améliorer la résistance à la dégradation (la rémanence) de l'hydrogel implanté, tout en conservant la possibilité d'injecter ledit hydrogel dans des conditions acceptables.

Pour améliorer l'efficacité de la réticulation, les inventeurs ont songé, dans un premier temps, à faire intervenir plus d'agent de réticulation. Cette approche a rapidement été écartée dans la mesure où elle entraîne inéluctablement une dénaturation du polymère en cause et une pollution chimique du produit réticulé obtenu.

Lesdits inventeurs ont alors songé à augmenter la concentration de polymère dans le mélange réactionnel. Cette seconde approche a, de la même façon dû, a priori, être écartée à la considération des polymères utilisés classiquement à ce jour : polymères de forte masse moléculaire. Ainsi, pour le hyaluronate de sodium, il est toujours utilisé à de fortes masses (Mw $\geq 10^6$ Da, $\approx 2.10^6$ Da, $3.10^6$ Da), à des concentrations voisines de la concentration maximale qui est d'environ 105-110 mg/g. L'utiliser à plus forte concentration est difficile (la viscosité du mélange réactionnel devient trop élevée) et entraîne inéluctablement des problèmes de solubilité, de mauvaise homogénéité

Concentrer le milieu réactionnel s'avère en revanche possible avec des polymères de faible masse moléculaire (du hyaluronate de sodium de masse moléculaire 300 000 Da, présentant une viscosité intrinsèque de 600 ml/g (l'homme du métier connaît parfaitement la relation entre ces deux paramètre masse moléculaire (M) et viscosité intrinsèque ($\eta$), relation donnée par la formule de Mark-Houwink : $M = k\,\eta^\alpha$, k et $\alpha$ présentant des valeurs qui dépendent de la nature du polymère en cause) peut être concentré de 110 à 200 mg/g). Malheureusement, le polymère réticulé obtenu génère dans ces conditions un hydrogel injectable non homogène, biphasique.

Dans un tel contexte, les inventeurs ont, de manière surprenante, établi qu'en associant polymère(s) de faible masse moléculaire et polymère(s) de forte masse moléculaire, on obtient un excellent compromis, à savoir la possibilité de générer, pour un taux de réticulation non excessif (équivalent à celui de l'art antérieur) un hydrogel monophasique, injectable avec des propriétés mécaniques et de rémanence améliorées. Cette association faible masse/forte masse permet d'obtenir un hydrogel répondant de manière plus que satisfaisante au cahier des charges ci-après :

- monophasique,
- propriétés mécaniques et rémanence meilleures que celles des produits équivalents de l'art antérieur,
- injectabilité non affectée, voire améliorée, restant possible avec des forces d'injection classiques au moyen de dispositifs d'injection classiques.

Le facteur clé du procédé de réticulation de l'invention réside donc dans la concentration des réactifs (qui est augmentée par rapport à celle des mélanges réactionnels de l'art antérieur, du fait de l'intervention de polymère(s) de faible masse moléculaire), la réticulation desdits réactifs concentrés étant toutefois "maîtrisée" par l'intervention de polymère(s) de forte masse moléculaire, qui garantissent l'homogénéité du produit réticulé puis de l'hydrogel obtenus.

Selon son premier objet, la présente invention concerne donc un procédé de réticulation d'au moins un polymère choisi parmi les polysaccharides et leurs dérivés, mis en oeuvre en solvant aqueux par action d'une quantité efficace et non excessive d'au moins un agent de réticulation, amélioré en ce qu'il est mis en oeuvre sur un mélange renfermant au moins un polymère de faible masse moléculaire et au moins un polymère de forte masse moléculaire.

Ledit mélange renferme bien entendu le(s)dit(s) polymère(s) de faible masse moléculaires en quantité suffisante pour garantir une concentration relativement élevée du milieu réactionnel en polymère(s) et le(s)dit(s) polymère(s) de forte masse moléculaire en quantité suffisante pour garantir une consistance homogène audit polymère réticulé obtenu.

Le procédé de réticulation de l'invention est un procédé de réticulation de polymères choisis parmi les polysaccharides et leurs dérivés. Le(s) polymère(s) en cause peu(ven)t donc être naturel(s) ou synthétique(s). Des exemples de polymères naturels sont l'acide hyaluronique et ses sels, d'autres glycosaminoglycanes, tels les sulfates de chondroïtine, le sulfate de kératine, l'héparine, le sulfate d'héparane, l'acide alginique et ses sels biologiquement acceptables, l'amidon, l'amylose, le dextrane, le xanthane, le pullulane... Des exemples de dérivés synthétiques de polysaccharides naturels sont la carboxycellulose, la carboxyméthylcellulose, des alkylcelluloses, telles l'hydroxyéthylcellulose, l'hydroxypropylméthylcellulose (HPMC), l'amidon oxydé...

Le procédé de invention est susceptible de convenir pour la réticulation de l'un quelconque de ces polymères, dans la mesure où ledit polymère intervient à des masses moléculaires faible(s) et forte(s).

Le procédé de l'invention est susceptible de convenir pour la réticulation de mélanges de tels polymères, lesdits mélanges renfermant au moins un polymère de faible masse moléculaire et au moins un mélange de forte masse moléculaire.

Les qualificatifs "faible" et "fort" des masses moléculaires en cause ne peuvent évidemment être plus précisément définis, à ce stade de la description de l'invention, dans la mesure où ils dépendent du mélange en cause, de la nature du(des) polymère(s) en présence. De la même manière, on ne peut, de manière générale, indiquer des proportions relatives d'intervention du(des) polymère(s) en présence. L'homme du métier a toutefois parfaitement compris l'esprit de l'invention. Il s'agit de concentrer le milieu réactionnel avec le(s) polymère(s) de faible masse moléculaire mais de faire toutefois intervenir au moins un polymère de forte masse moléculaire pour modérer, contrôler la réticulation en cause. On souhaite obtenir un produit réticulé cohérent, précurseur d'un hydrogel monophasique. On veut éviter la formation de grumeaux, éventuellement cohérents à l'issue de la réticulation mais susceptibles de perdre leur cohérence lors de la préparation de l'hydrogel injectable.

Les explications données ci-dessus le sont a posteriori. Le résultat obtenu n'était nullement prévisible.

Dans le cadre d'une variante avantageuse, le milieu réactionnel renferme un unique polymère, qui intervient à au moins deux masses moléculaires différenciées : au moins une faible et au moins une forte. Dans le cadre de cette variante avantageuse, le même polymère intervient de préférence à une unique faible masse moléculaire et à une unique forte masse moléculaire.

Le polymère en cause est avantageusement un sel de l'acide hyaluronique. Il est très avantageusement choisi parmi le sel de sodium, le sel de potassium et leurs mélanges. Il consiste de préférence en le sel de sodium (NaHA).

Dans le contexte de la réticulation de ce type de polymère, l'homme du métier comprend que ladite réticulation est mise en oeuvre dans un solvant aqueux basique. De manière générale, ladite réticulation est évidemment mise en oeuvre dans des conditions de pH favorable à la dissolution du polymère en cause.

Dans le contexte de la réticulation de ce type de polymère (sel(s) de l'acide hyaluronique), selon une variante préférée de mise en oeuvre de la réticulation, le mélange réactionnel renferme :

- au moins un sel de l'acide hyaluronique de faible masse moléculaire m, avec $m \leq 9,9.10^5$ Da, avantageusement $10^4$ Da $\leq m \leq 9,9.10^5$ Da ; et
- au moins un sel de l'acide hyaluronique de forte masse moléculaire M, avec $M \geq 10^6$ Da, avantageusement $10^6$ Da $\leq \underline{M} \leq 10^8$ Da, et très avantageusement $1,1.10^6$ Da $\leq M \leq 5.10^6$ Da ; lesdits sels de faible et forte masse moléculaire étant avantageusement de même nature, consistant très avantageusement en du hyaluronate de sodium (NaHA).

Dans un tel contexte, ledit mélange réactionnel a avantageusement une viscosité intrinsèque inférieure à 1900 ml/g, i.e. $\Sigma\omega_i[\eta_i]_0 < 1900$ ml/g, avec $\omega_i$ : fraction massique de la fraction i de polymère, présentant une viscosité intrinsèque $[\eta_i]_0$, dans le mélange réactionnel. L'homme du métier connaît le paramètre viscosité intrinsèque et n'ignore pas les lois d'additivité dudit paramètre.

La condition énoncée ci-dessus permet d'obtenir un hydrogel monophasique optimisé en référence à ses propriétés de rémanence et d'injectabilité. Elle fixe l'intervention relative des sels de faible (m) et forte (M) masse moléculaire.

Dans le contexte présentement évoqué (NaHA de masses moléculaires m et M), le mélange réactionnel renferme avantageusement plus de 50 %, très avantageusement plus de 70 %, en masse d'au moins un sel de l'acide hyaluronique de faible masse moléculaire m et donc, logiquement, avantageusement moins de 50 %, très avantageusement moins de 30 %, en masse d'au moins un sel de l'acide hyaluronique de forte masse moléculaire M.

On a, en général, pour l'obtention de l'effet escompté, au moins 5 % en masse d'au moins un sel de l'acide hyaluronique de forte masse moléculaire M dans le mélange réactionnel.

Le procédé de réticulation de l'invention est avantageusement mis en oeuvre avec du sel de sodium de l'acide hyaluronique, intervenant à <u>une</u> faible masse moléculaire m et à <u>une</u> forte masse moléculaire M. On a très avantageusement alors : $m \approx 3.10^5$ Da et $M \approx 3.10^6$ Da.

A titre d'agent de réticulation, avec tous types de polymère, on peut faire intervenir tout agent connu pour réticuler les polysaccharides et leurs dérivés, par l'intermédiaire de ses fonctions hydroxyles - agent réticulant au moins bifonctionnel, pour assurer la réticulation - et notamment un époxy ou ses dérivés.

On préconise l'intervention d'agents de réticulation, bifonctionnels, seuls ou en mélange. On préconise particulièrement l'intervention de l'épichlorhydrine, du divinylsulfone, du 1,4-bis(2,3-époxypropoxy)butane (ou 1,4-bisglycidyloxybutane ou encore 1,4-butanedioldiglycidyléther ou BDDE), du 1,2-bis(2,3-époxypropoxy)éthylène, du 1-(2,3-époxypropyl)-2,3-époxycyclohexane, d'aldéhydes tels le formaldéhyde, le glutaraldéhyde et le crotonaldéhyde, pris seuls ou en mélange. On préconise tout particulièrement l'intervention du 1,4-bis-(2,3-époxypropoxy)butane ou BDDE.

L'homme du métier saura déterminer la quantité efficace et non excessive d'agent(s) de réticulation à faire intervenir. On préconise d'utiliser une quantité efficace et non excessive telle qu'elle assure théoriquement un taux de réticulation ($\tau$), défini par le rapport

$$\tau = \frac{\text{Nombre total de fonctions réactives dudit agent de réticulation}}{\text{Nombre total de motifs disaccharides des molécules de polymère}} \times 100$$

compris entre 0,5 et 70 %, avantageusement entre 4 et 50 %.

Le procédé de réticulation de l'invention est original de par les formes d'intervention des polymères en cause. Il est sinon mis en oeuvre de façon classique avec au moins un agent de réticulation. On note que ledit agent de réticulation est généralement mis à réagir sur le(s) polymère(s) dissous. Il n'est toutefois nullement exclu qu'il intervienne sur le(s)dit(s) polymère(s) en cours d'hydratation, selon le procédé décrit dans WO-A-02 06 350.

Le réticulat obtenu à l'issue de la mise en oeuvre du procédé de réticulation de l'invention est généralement formulé pour générer l'hydrogel monophasique injectable recherché. Il est si nécessaire préalablement neutralisé. On a vu que la réticulation des sels de l'acide hyaluronique est en effet mise en oeuvre en milieu basique. La formulation est mise en oeuvre dans une solution tamponnée à un pH compatible avec le corps humain (puisque l'hydrogel en cause est généralement prévu pour une injection dans le corps humain) : pH compris entre 6,5 et 7,5 avantageusement entre 7 et 7,4, très avantageusement entre 7,1 et 7,3. Dans ladite solution, le polymère réticulé s'équilibre. Il acquiert également une osmolarité compatible avec celle du corps humain... De façon surprenante, à l'issue cette étape de formulation, les polymères réticulés de l'invention dilués sont des hydrogels <u>monophasiques</u>.

Selon une variante de mise en oeuvre préférée, on prépare un hydrogel injectable de l'invention, en réticulant un mélange d'au moins un polymère, sel(s) de l'acide hyaluronique (voir ci-dessus), en neutralisant le réticulat obtenu, puis en le formulant à une solution tamponnée à un pH compris entre 7,1 et 7,3, à une concentration comprise entre 10 et 40 mg/g, avantageusement entre 20 et 30 mg/g.

Le procédé de préparation de l'hydrogel monophasique injectable, à partir du polymère réticulé (selon le procédé de réticulation, premier objet de la présente invention) constitue le second objet de la présente invention.

On en vient aux troisième et quatrième objets qui consistent respectivement en le polymère réticulé susceptible d'être obtenu à l'issue de la mise en oeuvre du procédé de réticulation (premier objet) et l'hydrogel monophasique injectable susceptible d'être obtenu par formulation (second objet) dudit polymère réticulé, telle que précisée ci-dessus.

Lesdits polymère et hydrogel renferment avantageusement du hyaluronate de sodium de faible masse moléculaires et du hyaluronate de sodium de forte masse moléculaire ; ledit hyaluronate de sodium de faible masse moléculaire intervenant très avantageusement à plus de 50 % en masse.

La structure de l'hydrogel monophasique injectable - quatrième objet de la présente invention - est originale. Sa consistance résiste à la dégradation. Cette résistance de l'hydrogel est nettement supérieure à celle des produits équivalents de l'art antérieur.

L'homme du métier n'ignore pas qu'une des méthodes pour estimer la consistance d'un hydrogel, notamment de ce type, est la mesure du paramètre :

$$\tan.\mathrm{delta} = \frac{G''}{G'} = f(\text{fréquence de sollicitation}).$$

Les hydrogels de l'invention ont les débouchés indiqués dans l'introduction du présent texte. Ils s'y révèlent particulièrement performants.

On se propose maintenant d'illustrer l'invention sous ses différents aspects, par les exemples ci-après. On a plus précisément :

- l'exemple 1 qui illustre l'art antérieur (réticulation d'un polymère de forte masse moléculaire),
- l'exemple 2 qui illustre les propos énoncés dans l'introduction du présent texte (réticulation du même polymère de faible masse moléculaire),
- les exemples 3 et 4 qui illustrent l'invention (réticulation du même polymère intervenant à différentes quantités relatives à faible et forte masse moléculaire).

On précise préalablement quelques méthodes de mesure, utilisées pour caractériser les produits en cause.

**Mesure de la viscosité intrinsèque**

La viscosité intrinsèque du hyaluronate de sodium (NaHA) (en ml/g) est déterminée conformément à la Pharmacopée Européenne du NaHA (2.2.9), par un viscosimètre capillaire de type Ubelhode.

**Mesure de la force d'éjection (pas de norme spécifique sur ce test)**

L'injectabilité du gel à base de NaHA est déterminée par la mesure de la force nécessaire (en Newton, N) pour éjecter le gel contenu dans une seringue type, à travers une aiguille de 27 G½, à une vitesse de 12,5 mm/min. Les essais ont été effectués sur un appareil de traction Verstatet[®] commercialisé par la Société Mecmésin.

**Mesure de la rémanence**

La consistance du gel est caractérisée à 25°C par une mesure rhéologique des modules élastique (G') et visqueux (G") en fonction de la fréquence (de 0,05 à 10 Hz) et dans les domaines de déformation constante, au moyen d'un rhéomètre à contrainte imposée (Carrimed CSL 500 de TA Instruments) et d'une géométrie cône/plan 4 cm 2°. Ce rhéomètre est contrôlé et étalonné régulièrement. La dégradation du gel réticulé se traduit par une évolution de sa consistance, que l'on mesure par l'augmentation du paramètre tangente delta (tan.delta=G"/G') en fonction du temps, à la fréquence de 1 Hz. Les gels sont dégradés en étant portés à la température de 93°C. A cette température, on mesure le temps au bout duquel la tan.delta atteint la valeur 0,65 (valeur qui correspond à un état de gel dégradé). Arbitrairement, on a fixé pour le gel de l'exemple 1, un indice de rémanence de 1 (correspondant audit temps). Les valeurs d'indice de rémanence, indiquées pour les autres gels, sont des valeurs relatives.

## Aspect de l'hydrogel

### Monophasique

Aspect microscopique : pas de phase liquide apparente - fragmentation fine du gel en facettes

Aspect microscopique souple et filant

### Biphasique

Aspect macroscopique fragments de gels qui baignent dans un milieu liquide peu visqueux.

Aspect macroscopique "purgée" qui fragmente très facilement - pas de cohésion du gel ni d'aspect filant.

### EXEMPLE 1 : fibres de haute masse

On pèse 3,5 g de fibres de hyaluronate de sodium (NaHA), de viscosité intrinsèque = 2 800 ml/g, avec un taux d'humidité de 8,7 %, auquel on ajoute 25,6 g de NaOH à 0,25 N. L'hydratation des fibres dure 2 h, avec une homogénéisation manuelle régulière à la spatule. 0,96 g d'une solution de 1,4-butanedioldiglycidyléther (BDDE) dilué au 1/5ème dans la soude 0,25 N est ajouté au milieu réactionnel suivi d'une homogénéisation mécanique de 15 min avant immersion dans un bain thermostaté à 50°C $\pm$ 1°C.

$$R = [BDDE)_0/[NaHA]_0 = 6 \% ; [NaHA]_i = 105 \text{ mg/g}$$

La réaction dure 2 h. Le réticulat est neutralisé à pH = 7,2 dans une solution de tampon phosphate, puis est dialysé. L'hydrogel obtenu est alors ajusté en concentration ([NaHA]$_f$ = 26 mg/g) et homogénéisé mécaniquement avant d'être conditionné en seringues et stérilisé à l'autoclave par la chaleur humide.
Force d'injection après stérilisation : 25 N
Indice de rémanence de l'hydrogel 1,0
Hydrogel monophasique

### EXEMPLE 2 : fibres de faible masse

On pèse 1,56 g de fibres de hyaluronate de sodium (NaHA), de viscosité intrinsèque = 600 ml/g, avec un taux d'humidité de 5,5 %, auquel on ajoute 7,15 g de NaOH à 0,25 N. L'hydratation des fibres dure 2 h, avec une homogénéisation manuelle régulière à la spatule. 0,31 g d'une solution de 1,4-butanedioldiglycidyléther (BDDE) dilué au 1/5ème dans la soude 0,25 N est ajouté au milieu réactionnel suivi d'une homogénéisation mécanique de 15 min avant immersion dans un bain thermostaté à 50°C $\pm$ 1°C.

$$R = [BDDE]_0/[NaHA]_0 = 6,8 \% ; [NaHA]_i = 174 \text{ mg/g}$$

La réaction dure 2 h. Le réticulat est neutralisé à pH = 7,2 dans une solution phosphate, puis est dialysé. L'hydrogel obtenu est alors ajusté en concentration ([NaHA]$_f$ = 26 mg/g) et homogénéisé mécaniquement avant d'être conditionné en seringues et stérilisé à l'autoclave.
Force d'injection après stérilisation : 24 N
Indice de rémanence de l'hydrogel : 6,0
Hydrogel biphasique

**EXEMPLE 3 : mélange de fibres**

On pèse 0,763 g de fibres de hyaluronate de sodium (NaHA) de viscosité intrinsèque = 600 ml/g avec un taux d'humidité de 5,5 % et 0,237 g de fibres de hyaluronate de sodium de viscosité intrinsèque 2 800 ml/g, avec un taux d'humidité de 9,3 %. Proportion massique dans le mélange : 600/2 800 : 77/23 (w/w).
Le mode opératoire reste identique à celui de l'exemple 2.
R = $[DBBE]_0/[NaHA]_0$ = 7 % ; $[NaHA]_i$ = 140 mg/g ; $[NaHA]_f$ = 26 mg/g
Force d'injection après stérilisation : 15 N
Indice de rémanence de l'hydrogel : 3,6
Hydrogel monophasique

**EXEMPLE 4 : mélange de fibres**

L'expérience de l'exemple 3 est reproduite en modifiant la proportion massique. Proportion massique dans le mélange : 600/2 800 : 90/10 (w/w)
Le mode opératoire est identique à celui de l'exemple 2.
R = $[BDDE]_0/[NaHA]_0$ = 6,5 % ; $[NaHA]_i$ = 140 mg/g ;
$[NaHA]_f$ = 26 mg/g
Force d'injection après stérilisation 14 N
Indice de rémanence de l'hydrogel : 7,7
Hydrogel monophasique
Lesdits exemples sont récapitulés dans le tableau ci-après.

TABLEAU

| $[NaHA]_0$ = concentration en NaHA dans le milieu réactionnel à $t_0$ | | | | | | | | |
|---|---|---|---|---|---|---|---|---|
| $[NaHA]_f$ = concentration en NaHA dans l'hydrogel final après réaction et dilution avec une quantité qsp de tampon phosphate | | | | | | | | |
| G' : module élastique de l'hydrogel final (Pa.s) | | | | | | | | |
| G" : module visqueux de l'hydrogel final (Pa.s)           Rhéomètre Carrimed CSL 500 | | | | | | | | |
| Tan.delta = G"/G' | | | | | | | | |
| $\eta_{int.}$ : viscosité intrinsèque de la fibre de NaHA/Viscosimètre Ubelhode | | | | | | | | |
| F: force d'éjection du gel en N à travers une aiguille 27G½/Dynamomètre 100N | | | | | | | | |
| n° | $\eta_{in}$(ml/g) % = proportion massique dans le mélange | R= $m_{BDDE}/m_{NaHA}$ | $[NaHA]_0$ mg/g | $[NaHA]_f$ dans gel final mg/g | Aspect * | G', G", tan. delta (1 Hz) | $F_{ap\ ster}$ 27 G ½ | Indice de rémanenc e |
| 1 | (100%) 2 800 | 6% | 105 | 26 | M | 143/65/0,40 | 25 | 1 |
| 2 | (100%) 600 | 6,8 % | 174 | 26 | B | 1300/100/0,0 8 | 24 | 6 |
| 3 | (77%) 600+ (23% )2800 | 7 | 140 | 26 | M | 262/27/0,10 | 15 | 3,6 |
| 4 | (90%) 600+ (10% ) 2 800 | 6,5 | 140 | 26 | M | 571/41/0,07 | 14 | 7,7 |
| * M = monophasique B = biphasique | | | | | | | | |

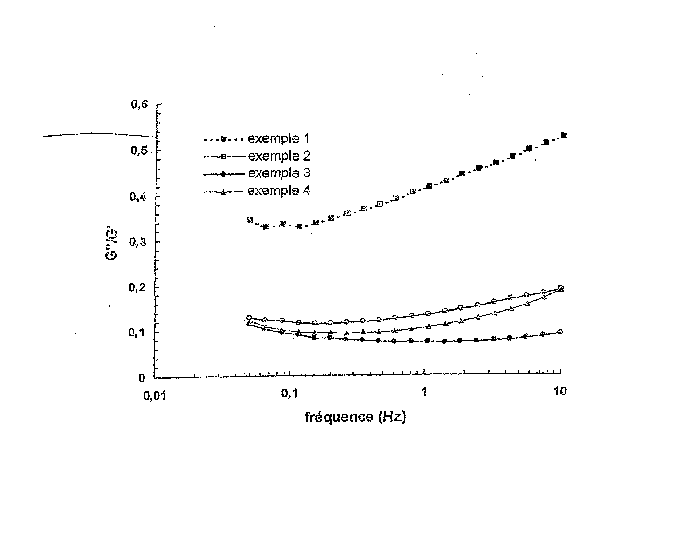

Sur la figure annexée, on a montré pour chacun des quatre hydrogels, préparés selon les exemples 1 à 4, les courbes :

Tan.delta = f (fréquence de sollicitation).

Les hydrogels de l'invention (exemples 3 et 4) ont un comportement rhéologique différent de celui de l'hydrogel de l'art antérieur (exemple 1).

Ils sont par ailleurs monophasiques et en cela très différents de l'hydrogel de l'exemple 2 (biphasique).

**Aspects préférs**

1. Procédé de réticulation d'au moins un polymère, choisi parmi les polysaccharides et leurs dérivés, mis en oeuvre en solvant aqueux par action d'une quantité efficace et non

excessive d'au moins un agent de réticulation, caractérisé en ce qu'il est mis en oeuvre sur un mélange renfermant au moins un polymère de faible masse moléculaire et au moins un polymère de forte masse moléculaire.

2. Procédé selon l'aspect 1, caractérisé en ce que ledit mélange renferme un unique polymère, présentant au moins deux masses moléculaires différentes : au moins une faible et au moins une forte ; présentant avantageusement deux masses moléculaires différentes : une faible et une forte.

3. Procédé selon l'un des aspects 1 ou 2, caractérisé en ce que ledit polymère est un sel de l'acide hyaluronique.

4. Procédé selon l'aspect 3, caractérisé en ce que ledit sel de l'acide hyaluronique est choisi parmi le sel de sodium, le sel de potassium et leurs mélanges ; et consiste avantageusement en le sel de sodium.

5. Procédé selon l'un quelconque des aspects 1 à 4, caractérisé en ce que ledit mélange renfermé :

- au moins un sel de l'acide hyaluronique de faible masse moléculaire m, avec $m \leq 9,9.10^5$ Da, avantageusement $10^4$ Da $\leq m \leq 9,9.10^5$ Da ; et

- au moins un sel de l'acide hyaluronique de forte masse moléculaire M, avec $M \geq 10^6$ Da, avantageusement $10^6$ Da $\leq M \leq 10^8$ Da, et très avantageusement $1,1.10^6$ Da $\leq M \leq 5.10^6$ Da;
lesdits sels de faible et forte masse moléculaire étant avantageusement de même nature, consistant très avantageusement en du hyaluronate de sodium.

6. Procédé selon l'aspect 5, caractérisé en ce que ledit mélange a une viscosité intrinsèque inférieure à 1 900 ml/g.

7. Procédé selon l'un des aspects 5 ou 6, caractérisé en ce que ledit mélange renferme plus de 50 %, avantageusement plus de 70 %, en masse d'au moins un sel de l'acide hyaluronique de faible masse moléculaire m et moins de 50 %, avantageusement moins de 30 % en masse d'au moins un sel de l'acide hyaluronique de forte masse moléculaire M.

8. Procédé selon l'un quelconque des aspects 5 à 7, caractérisé en ce que ledit mélange renferme au moins 5 % en masse d'au moins un sel de l'acide hyaluronique de forte masse moléculaire.

9. Procédé selon l'un quelconque des aspects 5 à 8, caractérisé en ce que ledit mélange renferme environ 90 % en masse de sel de sodium de l'acide hyaluronique présentant une masse moléculaire d'environ $3.10^5$ Da et environ 10 % en masse de sel de sodium de l'acide hyaluronique présentant une masse moléculaires d'environ $3.10^6$ Da.

10. Procédé selon l'un quelconque des aspects 1 à 9, caractérisé en ce que ledit agent de réticulation est choisi parmi les agents de réticulation bifonctionnels et leurs mélanges ; avantageusement choisi parmi l'épichlorhydrine, le divinylsulfone, le 1,4-bis(2,3-époxypropoxy)-butane, le 1,2-bis(2,3-époxypropoxy)éthylène, le 1-(2,3-époxypropyl)-2,3-époxycyclohexane, les aldéhyde tels le formaldéhyde, le glutaraldéhyde et le crotonaldéhyde, et leurs mélanges ; consiste très avantageusement en le 1,4-bis(2,3-époxypropoxy)butane.

11. Procédé selon l'un quelconque des aspects 1 à 10, caractérisé en ce que ladite quantité efficace et non excessive d'au moins un agent de réticulation assure théoriquement un taux de réticulation, défini par le rapport : 100 x (nombre total de fonctions réactives dudit agent de réticulation/nombre total de motifs disaccharidiques des molécules de polymère présentes), compris entre 0,5 et 70 %, avantageusement entre 4 et 50%.

12. Procédé de préparation d'un hydrogel monophasique injectable d'au moins un polymère réticulé choisi parmi les polysaccharides et leurs dérivés, caractérisé en ce qu'il comprend :

- la réticulation d'un mélange selon l'une quelconque des aspects 1 à 11,

- la formulation dudit mélange réticulé, neutralisé si nécessaire, dans une solution tamponnée à un pH compris entre 6,5 et 7,5, avantageusement compris entre 7 et 7,4, très avantageusement compris entre 7,1 et 7,3.

13. Procédé selon l'aspect 12, caractérisé en ce qu'il comprend :

- la réticulation d'un mélange selon l'une quelconque des aspects 3 à 11 ;

- la formulation dudit mélange réticulé, neutralisé, dans une solution tamponnée à un pH compris entre 7,1 et 7,3, à une concentration comprise entre 10 et 40 mg/g, avantageusement entre 20 et 30 mg/g.

14. Polymère réticulé susceptible d'être obtenu à l'issue de la mise en oeuvre d'un procédé de réticulation selon l'un quelconque des aspects 1 à 11.

15. Hydrogel monophasique injectable susceptible d'être obtenu à l'issue de la mise en oeuvre d'un procédé de préparation selon l'un des aspects 12 ou 13.

16. Hydrogel monophasique injectable selon l'aspect 15, renfermant, réticulé, du hyaluronate de sodium de faible masse moléculaire et du hyaluronate de sodium de forte masse moléculaire.

**Revendications**

1. Polymère réticulé avec un taux de réticulation défini par le rapport: 100 x (nombre total de fonctions réactives dudit agent de réticulation/nombre total de motifs disaccharidiques des molecules de polymère présentes), compris entre 0.5 et 70%, **caractérisé en ce que** le polymère contient un mélange de hyaluronate de sodium intervenant sous la forme de deux masses moléculaires différenciées renfermant:

- plus de 50% en masse de NaHA de faible masse moléculaire moyenne $10^4$ Da $\leq$ m $\leq$ 9.9 x $10^5$ Da déterminée à l'aide de la viscosité intrinsèque, et
- moins de 50%, mais au moins 5% en masse de NaHA de forte masse moléculaire moyenne M $\geq 10^6$ Da déterminée à l'aide de la viscosité intrinsèque.

2. Polymère réticulé selon la revendication 1, charactérisé en ce que le NaHA de faible masse moléculaire m a un masse moléculaire moyenne de m $\approx$ 3 x $10^5$ Da.

3. Polymère réticulé selon la revendication 1 ou 2, charactérisé en ce que le NaHA de forte masse moléculaire M a un masse moléculaire moyenne de M $\approx$ 3 x $10^6$ Da.

4. Polymère réticulé selon la revendication 1, 2 ou 3, **caractérisé en ce qu'**il renferme plus de 70% en masse d'au moins un NaHA de ladite faible masse moléculaire moyenne m et moins de 30 % en masse d'au moins un NaHA de ladite forte masse moléculaire moyenne M.

5. Polymère réticulé selon l'une quelconque des revendications 1 à 4, **caractérisé en ce qu'**il renferme environ 90 % en masse de NaHA présentant une masse moléculaire moyenne d'environ $3.10^5$ Da et environ 10 % en masse de NaHA présentant une masse moléculaire moyenne d'environ $3.10^6$ Da.

6. Polymère réticulé selon l'une quelconque des revendications 1 à 5, **caractérisé en ce que** ledit agent de réticulation est choisi parmi les agents de réticulation bifonctionnels et leurs mélanges.

7. Polymère réticulé selon la revendication 6, **caractérisé en ce que** ledit agent de réticulation est choisi parmi l'epichlorhydrine, le divinylsulfone, le 1,4-bis(2,3-époxypropoxy) butane, le 1,2-bis(2,3-époxypropoxy)éthylène, le 1-(2,3-époxypropyl)-2,3-époxycyclohexane, les aldéhyde tels le formaldéhyde, le glutaraldéhyde et le crotonaldéhyde, et leurs mélanges.

**8.** Polymère réticulé selon la revendication 6, **caractérisé en ce que** ledit agent de réticulation est le 1,4-bis(2,3-époxypropoxy) butane.

**9.** Polymère réticulé selon l'une quelconque des revendications 1 à 8, **caractérisé en ce que** l'agent de réticulation assure théoriquement un taux de réticulation, défini par le rapport: 100 x (nombre total de fonctions réactives dudit agent de réticulation/nombre total de motifs disaccharidiques des molécules de polymère présentes), compris entre 4 et 50 %.

**10.** Hydrogel monophasique injectable, comprenant le polymère réticulé selon la revendication 1 dans une solution tamponnée à un pH compris entre 6,5 et 7,5.

**11.** Hydrogel monophasique injectable selon la revendication 10 qui était neutralisée.

**12.** Hydrogel monophasique injectable selon la revendication 10 ou 11, caractérisé en a que la formulation dudit mélange réticulé, neutralisé si nécessaire, dans une solution tamponnée à un pH compris entre 7 et 7,4.

**13.** Hydrogel monophasique injectable selon la revendication 10 ou 11, caractérisé en a que la formulation dudit mélange réticulé, neutralisé si nécessaire, dans une solution tamponnée à un pH compris entre 7,1 et 7,3.

**14.** Hydrogel monophasique injectable selon la revendication 13, **caractérisé en ce qu'**il a:

- une concentration comprise entre 10 et 40 mg/g.

**15.** Hydrogel monophasique injectable selon la revendication 13, **caractérisé en ce qu'**il a une concentration comprise entre 20 et 30 mg/g.

**16.** Polymère réticulé susceptible d'être obtenu à l'issue de la mise en oeuvre d'un procédé de réticulation pour réticuler de hyaluronate de sodium (NaHA) en solvant aqueux par action d'une quantité efficace et non excessive d'au moins un agent de réticulation tel que ladite quantité assure théoriquement un taux de réticulation défini par le rapport: 100 x (nombre total de fonctions réactives dudit agent de réticulation/nombre total de motifs disaccharidiques des molecules de polymère présentes), compris entre 0.5 et 70%, **caractérisé en ce qu'**il est mis en oeuvre:

- sur un mélange de hyaluronate de sodium intervenant sous la forme de deux masses moléculaires différenciées renfermant:
- plus de 50% en masse de NaHA de faible masse moléculaire moyenne $10^4$ Da $\leq$ m $\leq$ 9.9 x $10^5$ Da déterminée à l'aide de la viscosité intrinsèque, et
- moins de 50%, mais au moins 5% en masse de NaHA de forte masse moléculaire moyenne M $\geq 10^6$ Da, déterminée à l'aide de la viscosité intrinsèque.

**17.** Polymère réticulé selon la revendication 16, qui est défini en outre par les caractéristiques selon l'une quelconque des revendications 2 à 9.

**18.** Hydrogel monophasique injectable susceptible d'être obtenu par formulation du polymère réticulé selon l'une des revendications 1 à 9, neutralisé si nécessaire, dans une solution tamponnée à un pH compris entre 6.5 et 7.5.

**19.** Hydrogel monophasique injectable selon al revendication 18, qui es défini en outre par les caractéristiques selon l'une quelconque des revendications 12 à 15.

Europäisches
Patentamt

European
Patent Office

Office européen
des brevets

**RAPPORT DE RECHERCHE EUROPEENNE**

Numéro de la demande

EP 14 20 0048

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 4 582 865 A (BALAZS ENDRE A  ET AL) 15 avril 1986 (1986-04-15) * exemples 1,2,10-13 * ----- | 1-19 | INV. C08B37/00 A61K47/36 A61L31/04 |
| X | US 4 886 787 A (DE BELDER ANTHONY N  ET AL) 12 décembre 1989 (1989-12-12) * colonne 4, ligne 38-52 * ----- | 1-19 | A61K6/097 A61L27/20 A61L27/50 |
| A | WO 94/09795 A1 (ALLERGAN INC [US]) 11 mai 1994 (1994-05-11) * revendications 13-20; exemples 1-3 * ----- | 1-19 | C08B37/08 C08J3/24 C08L5/08 |

A61L27/52

DOMAINES TECHNIQUES
RECHERCHES (IPC)

C08B
A61K
A61L
C08L

Le présent rapport a été établi pour toutes les revendications

| Lieu de la recherche | Date d'achèvement de la recherche | Examinateur |
|---|---|---|
| Munich | 25 mars 2015 | Gerber, Myriam |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons
...............................................................................
& : membre de la même famille, document  correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 14 20 0048

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

25-03-2015

| Document brevet cité au rapport de recherche | | Date de publication | Membre(s) de la famille de brevet(s) | | Date de publication |
|---|---|---|---|---|---|
| US 4582865 | A | 15-04-1986 | AU | 569157 B2 | 21-01-1988 |
| | | | AU | 572419 B2 | 05-05-1988 |
| | | | AU | 4304585 A | 12-06-1986 |
| | | | AU | 7217387 A | 27-08-1987 |
| | | | CA | 1230186 A1 | 08-12-1987 |
| | | | DE | 3520008 A1 | 19-06-1986 |
| | | | DE | 3546811 C2 | 02-09-1993 |
| | | | FR | 2574414 A1 | 13-06-1986 |
| | | | GB | 2168067 A | 11-06-1986 |
| | | | GB | 2181147 A | 15-04-1987 |
| | | | GB | 2181148 A | 15-04-1987 |
| | | | GB | 2205848 A | 21-12-1988 |
| | | | IT | 1187737 B | 23-12-1987 |
| | | | JP | H0430961 B2 | 25-05-1992 |
| | | | JP | H0637575 B2 | 18-05-1994 |
| | | | JP | H02138346 A | 28-05-1990 |
| | | | JP | S61138601 A | 26-06-1986 |
| | | | SE | 460792 B | 20-11-1989 |
| | | | US | 4582865 A | 15-04-1986 |
| US 4886787 | A | 12-12-1989 | CA | 1263647 A1 | 05-12-1989 |
| | | | DE | 3573785 D1 | 23-11-1989 |
| | | | EP | 0190215 A1 | 13-08-1986 |
| | | | JP | H0669490 B2 | 07-09-1994 |
| | | | JP | S61502729 A | 27-11-1986 |
| | | | SE | 456346 B | 26-09-1988 |
| | | | US | 4886787 A | 12-12-1989 |
| | | | WO | 8600912 A1 | 13-02-1986 |
| WO 9409795 | A1 | 11-05-1994 | AU | 5446094 A | 24-05-1994 |
| | | | US | 5492936 A | 20-02-1996 |
| | | | US | 6107347 A | 22-08-2000 |
| | | | WO | 9409795 A1 | 11-05-1994 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0466300 A
- WO 9633751 A
- WO 0001428 A
- WO 9835639 A
- WO 9835640 A
- US 4716154 A
- WO 0205753 A
- WO 0206350 A